# EUROPEAN PATENT APPLICATION

(11) **EP 3 249 388 A1**
(43) Date of publication of application: **29.11.2017**
(21) Application number: 16382225.7
(22) Date of filing: 23.05.2016
(51) Int. Cl.: G01N 21/65, A61B 5/00

(54) **METHOD FOR DIAGNOSING ALZHEIMER´S DISEASE**

(71) Applicant: Raman Health Technologies, S.L, 47151 Boecillo (ES)
(72) Inventor: VENEGAS DEL VALLE, Gloria, E-47151 Boecillo (ES); CATALÁ ESPÍ, Alejandro, E-47151 Boecillo (ES); ZANOTTI, Carlo, E-47151 Boecillo (ES)
(74) Representative: ABG Patentes, S.L.

(57) **Abstract**

A vibrational spectroscopy method for diagnosing Alzheimer's disease in a subject is disclosed, said method comprising the steps of a) determining in a Raman spectrum of a biofluid retrieved from said subject or in a sample derived from said biofluid the intensity of a Raman band around 1256 cm⁻¹; and b) comparing the intensity value obtained in step a) with a reference value, wherein an increase in the value of said Raman band intensity with respect to the reference value is indicative that the subject suffers Alzheimer's disease. Also disclosed are an apparatus for plasma sample analysis, a computer program, and a data carrier.

## Description

### FIELD OF THE INVENTION

The invention particularly relates to a method for diagnosing Alzheimer's disease based on vibrational spectroscopy.

### BACKGROUND OF THE INVENTION

There are over 30 million people living with Alzheimer's disease in Europe and more than 7 million people who have not been diagnosed.

Currently, Alzheimer's disease (AD) is diagnosed by a set of neuropsychological tests involving interviews with both patient and close relatives. This process involves subjective reasoning and requires significant expertise on the part of the clinician. However, these tests are not conclusive, since the symptoms of different types of dementia are easily misdiagnosed and may also be confused with the normal aging process.

Therefore, these tests should be complemented by diagnostic tests. Today there are mainly two methods for diagnosing AD and they are expensive, invasive and have multiple side effects. The positron emission tomography (PET) scans the patient's brain after being injected with a radioactive marker, which can cause severe allergic reactions. The other method analyzes the cerebrospinal fluid, which requires the patient to perform a lumbar puncture. Said method, relatively expensive, can cause severe headaches, temporary pain of numbness in the legs, infections and bleeding into the spinal canal.

There are some diagnostic methods based on the detection of β-amyloid in the cerebrospinal fluid. As mentioned above, this approach requires that patients undergo a lumbar puncture, which has many side effects. Other test analyses blood samples, but its usefulness has not been tested in patients over 80 years of age, and data have only be obtained from small group of patients.

Other blood tests as ABtest (Araclon Biotech) and INNO-BIA Aß (Innogenetics) are currently under development. These tests attempt to diagnose AD by measuring the concentration in blood plasma of a particular protein, β amyloid, which accumulates and causes damage in the brain in Alzheimer's disease. However, some results show that its concentration in blood plasma does not reflect the same in the brain.

Raman spectroscopy has been applied for the diagnosis of AD by means of measuring spectra in brain tissue sections, which resulted in the observation that sections from tissue brain in AD patients showed amyloid β peptide deposits, an increase in cholesterol and hyperphosphorylated tau (Archer et al. European Conference on Biomedical Optics (ECBO), Munich, Germany, 2007; Chen et al. Appl. Optics 48, 4743-4748 (2009)). However, this work does not provide specificity and sensitivity values and, in addition, requires the use of tissue samples, which are not as accessible as peripheral blood. Furthermore, changes occurring in brain tissue do not necessarily have to occur in peripheral blood as well.

Therefore, there is a need in the art for methods of diagnosing AD, in particular non- invasive and cheaper methods showing good accuracy.

### BRIEF DESCRIPTION OF THE INVENTION

In a first aspect, the invention relates to a vibrational spectroscopy method for diagnosing Alzheimer's disease in a subject comprising the following steps:
a) determining in a Raman spectrum of a biofluid from said subject or in a sample derived from said biofluid the intensity value of a Raman band at a frequency of around 1256 cm⁻¹; and
b) comparing the intensity value obtained in step a) with a reference value, wherein an increase in the value of said Raman band intensity with respect to the reference value is indicative that the subject suffers Alzheimer's disease.

In a second aspect, the invention relates to an apparatus for plasma sample analysis comprising:
a) a receptacle for receiving a plasma sample,
b) at least one Raman spectrometer
c) a computer system comprising means for implementing a diagnostic method according to the invention.

In a third aspect, the invention relates to a computer program comprising a code suitable for performing the diagnostic method according to the invention.

In a fourth aspect, the invention relates to a data carrier containing the computer program according to the invention.

In a fifth aspect, the invention relates to a therapy suitable for treating Alzheimer's disease for use in treating Alzheimer's disease in a subject wherein said subject has been selected according to a method according to the invention.

### DETAILED DESCRIPTION OF THE DRAWINGS

Figure 1. The figure shows the Raman spectral region where the band around 1256 cm⁻¹ appears.
Figure 2. The figure shows the difference in intensity of the band around 1256 cm⁻¹ between AD and healthy patients.
Figure 3. ROC curve associated with diagnosis of Alzheimer's disease based on the intensity of the band appearing at 1256 cm⁻¹.

### DETAILED DESCRIPTION OF THE INVENTION

The authors of the present invention have observed that the value of the intensity of a Raman band around 1256 cm⁻¹ is a spectroscopic parameter that allows the classification of patients with Alzheimer's disease with a high probability (see Example 1).

### Method for diagnosing Alzheimer's disease

In a first aspect the invention relates to a vibrational spectroscopy method for diagnosing Alzheimer's disease in a subject comprising the following steps:
a) determining in a Raman spectrum of a biofluid from said subject or in a sample derived from said biofluid the intensity value of a Raman band at a frequency of around 1256 cm⁻¹; and
b) comparing the intensity value obtained in step a) with a reference value,
wherein an increase in the value of said Raman band intensity with respect to the reference value is indicative that the subject suffers Alzheimer's disease.

"Diagnosing", as used herein, refers both to the process of attempting to determine and/or identify a possible disease in a subject, i.e. the diagnostic procedure, and to the opinion reached by this process, i.e. the diagnostic opinion. As such, it can also be regarded as an attempt at classification of an individual's condition into separate and distinct categories that allow medical decisions about treatment and prognosis to be made. As will be understood by those skilled in the art, the diagnosis of Alzheimer's disease, although preferred to be, need not be correct for 100% of the subjects to be diagnosed or evaluated. The term, however, requires that a statistically significant portion of subjects can be identified as suffering Alzheimer's disease. Whether a subject is statistically significant can be determined without further ado by the person skilled in the art using various well known statistic evaluation tools, e.g., determination of confidence intervals, p-value determination, Student's t-test, Mann-Whitney test, etc. Details are found in Dowdy and Wearden, Statistics for Research, John Wiley & Sons, New York 1983. Preferred confidence intervals are at least 50%, at least 60%, at least 70%, at least 80%, at least 90%, or at least 95%. The p-values are, preferably, 0.05, 0.01, 0.005 or lower.

The term "Alzheimer's disease" or "AD" or "Alzheimer's", as used herein, refers a mental deterioration associated with specific degenerative brain disease that is characterized by senile plaques, neuritic tangles and progressive neuronal loss which manifests clinically in progressive memory deficits, confusion, behavioural problems, inability to care for oneself, gradual physical deterioration and, ultimately, death. Typically, patients suffering from AD are identified using the NINCDS-ADRDA (National Institute of Neurological and Communicative Disorders and the Alzheimer's Disease and Related Disorders Association) criteria: Clinical Dementia Rating (CDR) = 1; Mini Mental State Examination (MMSE) = 16-24 points and Medial temporal atrophy (determined by Magnetic Resonance Imaging, MRI) >3 points in Scheltens scale.

As used herein, the term AD is intended to include all the stages of the disease, including, without limitation, the following stages defined by NINCDS-ADRDA Alzheimer's Criteria for diagnosis in 1984 (McKhann et al., 1984, Neurology 34:939-44):
- Definite AD: The patient meets the criteria for probable AD and has histopathological evidence of AD via autopsy or biopsy.
- Probable or prodromal AD: Dementia has been established by clinical and neuropsychological examination. Cognitive impairments also have to be progressive and be present in two or more areas of cognition. The onset of the deficits has been between the ages of 40 and 90 years and finally there must be an absence of other diseases capable of producing a dementia syndrome.
- Possible or non-prodromal AD: There is a dementia syndrome with an atypical onset, presentation or progression; and without a known etiology; but no co-morbid diseases capable of producing dementia are believed to be in the origin of it.

Additionally, the National Institute on Aging and the Alzheimer's Association revised in 2011 the diagnostic criteria into the NIA-AA's Criteria for Alzheimer's disease (Albert et al., 2011, Alzheimer's Dement 7:270-9; Jack et al., 2011, Alzheimer's Dement 7:257-62; McKhann et al., 2011, Alzheimer's Dement 7:263-9; Sperling et al., 2011, Alzheimer's Dement 7:280-92). The term AD is also intended to include the stages defined by these criteria.

"Vibrational spectroscopic method" as used herein, relates to the analysis of molecular properties based on vibrations at the molecular level. In particular, Raman is a scattering/dispersive technique effective in the 50 - 4,000 cm⁻¹ range and involves a shifting of incident light (Raman shift).

"The term "subject" or "individual" or "animal" or "patient" includes any subject, particularly a mammalian subject. Mammalian subjects include humans, domestic animals, farm animals, and zoo or pet animals such as dogs, cats, guinea pigs, rabbits, rats, mice, horses, cattle, cows, and so on. In a preferred embodiment, the subject is a human.

In a first step, the diagnostic method of the invention comprises determining in a Raman spectrum of a biofluid from the subject or in a sample derived from said biofluid the intensity value of a Raman band at a frequency of around 1256 cm⁻¹.

In the scope of the present invention, the expression "Raman spectrum" refers to collection of wavenumbers comprising the spectral region comprised between 600 and 3100 cm ⁻¹, although the spectral region may be broader provided it comprises the said previous spectral region.

The term "biofluid", as used herein, refers to any bodily fluid or liquid derived from the body of a living organism. Suitable samples for use in the diagnostic method of the invention include any bodily fluid selected from the group consisting of blood, serum, plasma, CSF, saliva, gingival crevicular fluid, urine, seminal plasma, tears, and nipple fluid.

In a preferred embodiment the biofluid sample is plasma.

As it is understood in the invention, "plasma" refers to the liquid element of blood, representing 60% of the total blood volume and lacking red and white blood cells. The plasma fraction is preferred over the platelet fraction because it is easier to obtain, besides the fact that the difficulties in obtaining platelets due to platelet aggregation or rupture phenomena are well-known.

The present invention can be carried out on substantially platelet-free plasma. Alternatively, a platelet-rich plasma sample is preferred in another aspect. As it is used in the present invention, "platelet-rich plasma" refers to plasma that has been enriched with platelets and generally contains more than 300-350,000 platelets/µL. Various methods known in the state of the art can be used for obtaining platelet-rich plasma. The various methods consist of obtaining blood in tubes with anticoagulant and subjecting them to different centrifugation conditions according to the different protocols, such that the blood separates into its basic components depending on density, selecting that fraction corresponding to the platelet-rich plasma.

According to the invention, the blood plasma fraction to be analyzed can be obtained previously from a human blood sample (preferably human peripheral blood) already obtained by means of any of the standard blood fractionation processes, such as centrifugation-filtration fractionation for example.

In a preferred embodiment, the diagnostic method of the invention is preceded by a step comprising recording a Raman spectrum of a biofluid from the subject or of a sample derived from said biofluid. In a more preferred embodiment, the biofluid sample is processed prior to the recording of the Raman spectrum. In a yet more preferred embodiment, the processing is carried out by dehydration. In a preferred embodiment, the dehydration is carried out by evaporation. In another embodiment, dehydration is carried out until dryness.

"Sample derived from the biofluid", as used herein relates to any sample derived from the processing of the biofluid. In a preferred embodiment, the sample derived from the biofluid is a dehydrated biofluid.

"Dehydrated biofluid", as used herein relates to a sample of biofluid that shows a removal of water content. In a preferred embodiment, the removal of water is complete.

In another preferred embodiment, the dehydrated biofluid is dehydrated plasma.

In another preferred embodiment, the sample is crystalized.

In a preferred embodiment, the dehydrated plasma is prepared following a process comprising extending a volume of between 6 and 100 µL of a blood plasma fraction on a CaF₂ or ZnSe crystal or on a metallic support and leaving it to completely evaporate to dryness at a temperature comprised between 10 and 25°C, preferably between 15 and 25°C.

The skilled person knows that the volume of sample will depend on the type of support, for example CaF₂ or ZnSe crystal or metallic support. In a preferred embodiment the volume of blood plasma fraction is between 6 and 14 µL or between 10-100 µL. In a more preferred embodiment, the volume is between 10-20 µL, 20-30 µL, 30-40 µL, 40-50 µL, 50-60 µL, 60-70 µL, 70-80 µL, 80-90 µL or 90-100 µL. In a yet more preferred embodiment, the volume of a blood plasma fraction is 10 µL.

In another preferred embodiment, the surface of the crystal or metallic support is between 0.5 to 2 cm², preferably 1 cm².

In another preferred embodiment, the CaF₂ crystal is used.

"CaF₂ crystal", as used herein relates to a cubic crystal with a single first order Raman line of 322 cm⁻¹ at 300°K, It shows high transmittance in the far UV to mid IR range, low refractive index, high chemical resistance and high laser damage threshold.

"ZnSe crystal", as used herein relates to a chemically inert, non-hygroscopic and highly pure product that is very effective in many optical applications due to its extremely low bulk losses, high resistance to thermal shock and stability in virtually all environments, easily machined

In another preferred embodiment a metallic support is used, preferably the metal being Ag, Au and Cu. Preparation of the surface can be through electrochemical roughening, metallic coating of a nano-structured substrate, or deposition of metallic nanoparticles.

The evaporation to dryness of the volume of said fraction can be performed at a temperature comprised between 10 and 25°C without observing any alteration of the samples for the purpose of the present invention. Evaporation is preferably performed between 15 and 25°C, both limits being included, for the purpose of faster sample evaporation.

Generally and in a non-limiting manner, when a non-fractionated human blood sample is initially used, the blood fraction sample (preferably a blood plasma sample) suitable for Raman spectroscopic analysis of the present invention can be prepared prior to acquiring the spectroscopic data according to a process comprising the following steps:
a. obtaining said blood fraction (preferably the blood plasma fraction) from a previously obtained human peripheral blood sample by means of any of the known centrifugation-filtration blood fractionation processes normally used;
b. extending the plasma sample on a 0.5 to 2 cm², preferably 1 cm² square surface CaF₂ or ZnSe crystal or metallic support;
c. completely evaporating to dryness a volume of the previous fraction, approximately 10 µL, to obtain 1µg of a dry solid residue of said fraction, where the evaporation of said fraction volume is performed at relatively low temperatures, specifically in the temperature range comprised between 10 and 25°C, more preferably between 15 and 25°C, said limits being included.

The term "spectra value", as used herein, refers to any measurable parameter that can be obtained from a spectrum and that can be used to compare several spectra. In the present invention, the spectral value is the intensity value.

The term "intensity value" refers to the intensity of a peak at a given wavenumber.

In the scope of the present invention, the expression "band around a frequency value expressed in cm-¹" refers to the band in the Raman vibrational spectrum whose intensity is obtained in the frequency range determined by vi ± X cm ⁻¹, wherein X is a variable frequency value that is at least 1, at least 2, at least 3, at least 4, at least 5, at least 6, at least 7, at least 8, at least 9, at least 10 cm⁻¹ or greater. In a preferred embodiment, X has a value of 2. Therefore a band around a frequency value of 1256 cm⁻¹ refers to the maximum band that is obtained in the 1254 to 1258 cm⁻¹ range. In the present specification, said term can also be referred to as "band approximating a frequency value expressed in cm⁻¹". The increase of ± X cm⁻¹ in a band frequency is indicated to define a frequency range amplitude of one and the same band which can be measured in various spectrometers differing in the frequency measurement accuracy. Therefore, it must be understood that the frequency values vi appearing in this specification can vary by ± X cm⁻¹ with respect to said frequency value depending on the spectrometer used.

Accordingly, the term "Raman intensity obtained around a frequency value vᵢ expressed in cm⁻¹" is understood to refer to the intensity value of the Raman spectrum obtained at the maximum of the band appearing in the frequency range determined by vᵢ ± X cm⁻¹. In the event that X has a value of 2, a Raman intensity obtained around a frequency value of 1256 cm⁻¹ corresponds to the intensity value of the maximum obtained in the frequency range comprised between 1254 cm⁻¹ and 1258 cm⁻¹.

In order to obtain the spectral value, the method according to the present invention contemplates normalization and deconvolution of the spectra.

A person skilled in the art will understand that, according to the method of the invention, in a preferred embodiment, the invention preferably contemplates correcting the baseline of the Raman spectrum and normalizing said spectrum with respect to its whole area in order to determine the intensity value.

The term "normalization", as used herein refers to the elimination of systematic differences among measurements. One of the most significant sources of systematic error in Raman spectral measurements arises from total intensity variations. Normalization may be useful to avoid that the differences in the intensity value are due to differences in the amount of sample assayed or due to variability in the quality of the sample used as well as to remove unwanted sources of systematic variation in Raman measurements, and the like.

A person skilled in the art will understand that the method of the invention also contemplates the normalization of the Raman spectrum. The normalization can be carried out using any band that does not change between AD patients and patients not suffering from AD. In a preferred embodiment, normalization can be carried out by dividing the spectrum value of the peak of interest by the value of the complete spectrum. Preferably the complete spectral region is comprised between the wavelength of 600 and 3100 cm ⁻¹.

It will be understood that in order to obtain the intensity value any of the normalization values mentioned above is used. The reference value is preferably normalized using the same normalization value. For instance, if the reference value is the intensity of the Raman band at a frequency of around 1256 cm⁻¹ in a control patient (i.e, a patient not suffering from AD), then the intensity of the band from the control spectrum has to be obtained by normalizing in the same manner as the intensity of the band of the sample under study. In this case, the reference value to be used in step c) to determine whether the intensity of the band is increased is the normalized reference value.

In a preferred embodiment, the intensity value is determined after normalizing the spectrum value with respect to the total area under the Raman curve.

The term "area", as used herein, refers to one the area defined by a peak of the spectrum which can be calculated by drawing a baseline across the peak and measuring the area enclosed in the peak. The baseline is typically drawn based on the points before and after the peak on the spectrum. In one embodiment, the area used for normalization is the area of the spectral region comprised between 600 and 3100 cm ⁻¹,

The term "deconvolution", as used in the present invention, refers to a method whereby the spectrum is resolved into its constituent elements, thereby allowing the identification of signals which would otherwise remain masked.

In order to obtain better results with the method described in the present invention, it is convenient to remove fluorescence signals generated in the spectrum by biological substances contained in the blood samples which at least partially mask the Raman spectra of the samples in question. Though fluorescence of the Raman spectra generated with Raman excitation with laser lines in the visible spectrum can be minimized by means of optimizing the microspectrometer to be used, the fluorescence suppression cannot be 100%. Thus, it is convenient to obtain the spectrum by means of using laser lines with near-infrared excitation, such as a 1064 nm neodymium-YAG laser line for example.

In a preferred embodiment, the Raman spectrum is recorded using near-infrared laser excitation line 750-1400 nm.

The second step of the method of the invention comprises comparing the Raman spectral value obtained in step a) with a corresponding reference spectral value.

As used herein reference spectral value relates to a laboratory value used as a reference for the values/data obtained from samples. The reference value (or reference level) can be an absolute value, a relative value, a value which has an upper and/or lower limit, a series of values, an average value, a median, a mean value, or a value expressed by reference to a control or reference value. A reference value can be based on the value obtained from an individual sample, such as, for example, a value obtained from a sample of study but obtained at a previous point in time. The reference value can be based on a high number of samples, such as the values obtained in a population of samples or based on a pool of samples including or excluding the sample to be tested. In a yet more preferred embodiment, when the reference sample is obtained from a pool of samples, then the samples are of the same type of sample as the sample obtained from the subject under study, i.e. if the Raman spectrum of the subject under study has been obtained from plasma, then the reference value is obtained from a Raman spectrum obtained from a pool of plasma samples.

In a preferred embodiment, the reference spectral value is the spectral value of a sample obtained from a control patient, more particularly from a subject not suffering Alzheimer's disease, and more preferably from a healthy subject. In a yet more preferred embodiment, the reference sample obtained from a control patient as defined above is the same type of sample as the sample obtained from the subject under study, i.e. if the Raman spectrum of the subject under study has been obtained from plasma, then the reference value is obtained from a Raman spectrum obtained from a plasma sample in the control subject. In another embodiment, the reference spectral value is the spectral value of a sample obtained from the patient under study but obtained at a previous time point and, in particular, at a time point when the patient did not show any symptoms of the disease. In a yet more preferred embodiment, the reference sample obtained from a patient at a previous time point as defined above is the same type of sample as the sample obtained from the subject under study, i.e. if the Raman spectrum of the subject under study has been obtained from plasma, then the reference value is obtained from a Raman spectrum obtained from a plasma sample from the same subject at a previous time point.

According to the method of the invention, an increase in the value of said Raman band intensity is indicative that the subject suffers Alzheimer's disease.

The value of a Raman band intensity is considered "increased", wherein the value of a Raman band intensity of a band around 1256 cm⁻¹ compared to the corresponding reference spectral value is at least at least 1.5%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%: at least 85%, at least 90%, at least 95%, at least 100%, at least 110%, at least 120%, at least 130%, at least 140%, at least 150% or more.

### Apparatus for plasma sample analysis

In another aspect, the invention relates to an apparatus for plasma sample analysis comprising
a) at receptacle for receiving a plasma sample,
b) at least one Raman spectrometer
c) a computer system comprising means for implementing a diagnostic method according to the invention

The devices forming the Raman spectrometer are known in the state of the art, basically a laser and a CCD (charge-coupled devices). The equipment can be formed by only one light source, or by two or more interchangeable monochromatic light sources (for example He-Ne laser Ar laser and laser diode) connected to an excitation optical fiber guiding the light to the optical head and the latter focusing the light on the samples. The light scattered by the sample is collected through the same optical head and by means of the collection optical fiber is guided to the monochromator which spatially and spectrally separates it. The CCD detects the signal diffracted by the monochromator and transforms the scattered light photons into a digital electric signal and the spectrum is forwarded to a computer.

Alternatively in the case of a dispersive spectrometer, the light is directed through the collimating optics to a diffraction grating , which disperses the light in order to project the spectrum on the CCD.
The purpose of the computer system of the apparatus of the invention is, among others, to control the acquisition of the scattered Raman signal and its subsequent analysis, including the mathematical processing. The computer system also performs control of the parameters of the CCD, the focused system of the laser beam and its power, among others. The computer system can additionally allow a certain degree of manipulation of the spectra, such as for example correction of the baseline, comparison between several spectra, calculations of areas and Fourier transforms, comparison of the spectral value of the sample to be analyzed with the reference spectral value or multivariate analysis comparison with values that allow differentiating between a blood sample associated with Alzheimer's disease and a blood sample associated with Alzheimer's disease, among others.

In another particular embodiment, the apparatus of the invention additionally comprises a microscope. In another preferred embodiment, the apparatus of the invention additionally comprises a video microscope having a beamsplitter that allows directing the light to both a webcam and the Raman head. In order to focusing the laser and collimating the Raman light an objective of a miscroscope is used.

The terms described in detail in the context of the diagnostic method of the invention and their definitions and particularities apply equally to this aspect of the invention.

### Computer program and data carrier

In another aspect, the invention relates to a computer program comprising a code suitable for performing the diagnostic methods according to the invention.

In another aspect, the invention relates to a data carrier containing the computer program according to the invention.

In another aspect, the invention relates to a computer system provided with means for implementing the diagnostic method according to the invention. The computer system can include:
(a) at least one memory containing at least one computer program suitable for controlling the operation of the computer system for implementing a method including: (i) receiving Raman spectra data about the sample from the patient and (ii) assigning the patient as a healthy subject or as an Alzheimer's disease patient based on the degree of identity between the spectrum obtained from the sample from the subject and the spectrum of the reference subject and
(b) at least one processor for running the computer program.

Another aspect of the present invention relates to a computer program for controlling a computer system for performing the steps according to the method of the invention.

The computer system can include one or more general processors or processors having particular purposes and associated memory, including volatile and non-volatile memory devices.

The machine-readable physical storage media useful in several embodiments of the invention can include any machine-readable physical storage medium, for example solid state memory (such as flash memory), machine-readable magnetic and optical storage media and devices, and memory using other persistent storage technologies. In some embodiments, a machine-readable medium can be any tangible medium which allows the computer to access computer programs and data. The machine-readable media can include erasable or non-erasable tangible volatile and non-volatile media implemented in any method or technology that can store information, such as machine-readable instructions, program modules, programs, data, data structures, and database information. In some embodiments of the invention, machine-readable medium includes but is not limited to RAM (Random Access Memory), ROM (Read-only Memory), EPROM (Erasable Programmable Read-only Memory), EEPROM (Electrically Erasable Programmable Read-only Memory), flash memory or other memory technology, CD-ROM (Compact Disc Read-only Memory), DVD (Digital Versatile Discs) or other optical storage medium, magnetic cassettes, magnetic tape, magnetic disc storage or other magnetic storage medium, other types of volatile and non-volatile memory, and any other tangible medium that can be used to store information and can be machine-read, including any suitable combination of the foregoing.

The present invention can be implemented in an autonomous computer or as part of a network computer system.

In some embodiments of the present invention, the Raman spectra used as reference can be used to record, register and retrieve electronically or digitally.

In some embodiments of this aspect and all the other aspects of the present invention, the system can compare the data in a "comparison module" which can use a variety of software formats and programs available for operative comparison for comparing spectra determined in the determination module with reference data. In another embodiment, the comparison module is configured for using pattern recognition techniques for comparing information of sequences of one or more inputs with one more reference data patterns. The comparison module can be configured to use existing commercially available or disposable software for comparing patterns and can be optimized for particular data comparisons that are performed.

In some embodiments, the comparison module provides a machine-readable comparison result that can be processed in the machine-readable form by means of predefined criteria, or user-defined criteria, to provide a report comprising content based in part on the comparison result that can be stored and accessed as required by a user using a display module. In some embodiments, a display module allows displaying content based in part on the comparison result for the user, wherein the content is a report indicative of the results of the comparison of the spectrum obtained from the sample from the patient of interest with the spectrum of a healthy subject.

In some embodiments, the display module allows displaying a report or content based in part on the comparison result for the end user, wherein the content is a report indicative of the results of the comparison of the spectrum of the patient with the reference spectrum. In some embodiments of this aspect and all other aspects of the invention, the comparison module, or any other module of the invention, can include an operating system (for example, UNIX, Windows) in which a relational database management system, a World Wide Web application and a World Wide Web server are run. The World Wide Web application can include the executable code necessary for generating database language instructions [for example, standard query language (SQL) instructions].

The computer instructions can be implemented in software, firmware or hardware and include any type of programmed step undertaken by modules of the information processing system. The computer system can be connected to a Local Area Network (LAN) or a Wide Area Network (WAN).

In some embodiments of this aspect and all other aspects of the present invention, a comparison module provides machine-readable data that can be processed in machine-readable manner by means of predefined criteria, or user-defined criteria, to provide retrieved content that can be stored and accessed as required by a user using a display module.

According to some embodiments of the invention, the computerized system can include or be operatively connected to a display module, such as a computer monitor, a touch screen or video display system. The display module allows presenting the user instructions to the system user, allowing the user to see system inputs and allowing the system to show the results to the user as part of a user interface. The computerized system can optionally include or be operatively connected to a printing device to make printed copies of the information outputted by the system.

In some embodiments of the present invention, a World Wide Web browser can be used to provide a user interface for allowing the user to interact with the system to enter information, to make requests and to show the retrieved content. Furthermore, the various functional modules of the system can be adapted for using a web browser to provide a user interface. By using a web browser, a user can make requests to retrieve data from data sources, such as databases, and interact with the comparison module to make comparisons and pattern matching. The user can indicate and click on user interface elements such as buttons, drop-down menus, displacement lines, etc., conventionally used in graphical user interfaces for interacting with the system and making the system perform the methods of the invention. The requests made with the user web browser can be transmitted in a network to a Web application which can process or format the request to conduct a query in one or more databases that can be used to provide the relevant information relating to the spectra generated, the content retrieved, process this information and generate the results.

The terms described in detail in the context of the diagnostic method of the invention and their definitions and particularities apply equally to this aspect of the invention.

### Personalized therapy

In another aspect, the invention relates to a therapy suitable for treating Alzheimer's disease for use in treating Alzheimer's disease in a subject wherein said subject has been selected or diagnosed according to a method according to the invention.

Alternatively, the invention relates to a method of treating Alzheimer's disease in a subject comprising administering a compound suitable for treating Alzheimer's disease to the subject in need thereof, wherein said subject has been selected according to a method according to the invention.

Alternatively, the invention relates to the use of a compound suitable for treating Alzheimer's disease for the preparation of a medicament for the treatment of a subject in need thereof, wherein said subject has been selected according to a method according to the invention.

"Therapy (compound) suitable for treating Alzheimer's" comprises any drug or treatment that allows treating Alzheimer's, i.e., a treatment for reducing, improving or eliminating symptoms of AD.

In a preferred embodiment, the therapy suitable for treating Alzheimer's disease is selected from the group consisting of a cholinesterase inhibitor, an N-methyl-D-aspartate (NMDA) receptor antagonist, anti β-amyloid immunotherapy, a gamma-secretase inhibitor and an anti-tau therapy.

"Cholinesterase inhibitor" refers to a chemical compound inhibiting the cholinesterase or anticholinesterase, preventing the destruction of the released acetylcholine, thereby causing an increase in the concentration and in the duration of the effects of the neurotransmitter. The two types of cholinesterase are acetylcholinesterase (ACHE) and butyrylcholinesterase (BCHE). The full cholinesterase sequence in humans has accession number P06276 in the Uniprot database (20 January 2016).

In a preferred embodiment, the compound used according to the invention is an acetylcholinesterase inhibitor.

There is evidence from pre-clinical studies and some studies in human beings that cholinesterase inhibition affects basic processes that have been involved in the pathogenesis of AD. For example, the acetylcholinesterase (AChE) inhibition can affect the expression of AChE isoforms and increase the expression of nicotine receptors, both being correlated with cognitive improvements in patients with AD. AChE inhibition has also been shown to affect amyloid precursor protein (APP) processing and attenuate the toxicity induced by Aβ by means of mechanisms including the interruption of Aβ production, the alteration of Aβ 1-40 and Aβ 1-42 levels and the formation of the soluble form of the amyloid precursor protein. Therefore, by way of non-limiting illustration, therapies suitable for treating mild to moderate AD, comprise cholinesterase inhibitors such as Cognex® (tacrine), Aricept (donepezil), Exelon® (rivastigmine) or Razadine® (galantamine), among others. The moderate to severe AD can be treated with Namenda® (memantine), which acts as NMDA antagonist receptors. Since NMDA antagonists work differently from those of cholinesterase inhibitors, both types of medicaments can be prescribed in combination. These medicaments can help in delaying or preventing symptoms of AD from worsening. The therapies according to the present invention additionally include music therapy, physical therapy, psychomotor education, occupational therapy or therapy with animals, among others.

Three cholinesterase inhibitors for treating Alzheimer's disease, are currently sold worldwide, specifically donepezil, galantamine and rivastigmine. Donepezil (1-benzyl-4-[(5,6-dimethoxy-1-indanon)-2-yl]-methyl-piperidine) is a reversible acetylcholinesterase (AChE) inhibitor. Galantamine([4aS-(4aa,6β,8aR*)]-4a,5,9,10,11,12-hexahydro-3-methoxy-11-methyl-6H-benzofuro[3a,3,2-ef] benzace pin-6-ol) is an alkoid isolated from snowdrop, Galanthus nivalis. It is a highly selective, reversible and competitive acetylcholinesterase inhibitor. Rivastigmine ((S)-N-ethyl-3-[1-(dimethylamino)ethyl]-N-methyl-phenyl-carbamate) is a reversible, non-competitive acetylcholinesterase and butyrylcholinesterase inhibitor.

Clinical and/or pre-clinical trials for other cholinesterase inhibitors, specifically for tacrine hydrochloride (CI-970, THA.HCl), huperzine A, acotiamide, dimebolin, DEBIO 9902, IN 101, phenserine tartrate, R-phenserine, stacofylline hydrochloride (S-9977, S-9977-2), NP-61, bisnorcymserine, COL-204, SPH 1371, SPH 1373, SPH 1375, SP 04, CM 2433, metrifonate, 7-methoxytacrine (7 MEOTA), P 11149, Arisugacin, FR 152558, HUP 13, isovanihuperzine A (IVHA), MHP 133, NP 7557, P 10358, P 11012, physostigmine salicylate, velnacrine maleate (HP-029, P83-6029A), epastigmine tartrate (L-693487), ipidacrine, zanapezil, ganstigmine, icopezil maleate (CP-118954, CP-118954-11), KW 5092, quilostigmine (HP-290, NXX-066), SM 10888, T 82, TAK 802, zifrosilone MDL-73745), BGC 201259, CHF 2060, CI 1002, E 2030, ER 127528, ET 142, F 3796, huprine X, MF 247, MF 268 bitartrate, MF 8615, P 26, PD 142012, RO 465934, SS 20, thiatolserine, tolserine tartrate, UR 1827. (ADIS R&D Insight, the 25/02/2010), have also been conducted and the foregoing could be used for treating AD according to the invention.

Additional compounds for inhibiting cholinesterases have been described, for example edrophonium, demecarium, ambenonium, neostigmine bromide, dehydroevodiamine chloride, eseroline, imperatorin, scopoletin (SCT), huperizine A (Hup A), heptylstigmine tartrate (MF-201), suronacrine maleate (HP-128), UCB-1 1056, berberine iodide, norpyridostigmine, quilostigmine (HP-290, NXX-066), THB-013, PD-142676, terestigmine tartrate (CHF-2060), thiacymserine, MF-8615, MF-268 bitartrate, anseculin hydrochloride (KA-672.HCl), ensaculin hydrochloride, icopezil maleate (CP-118954), eserine salicylate, physostigmine salicylate, JWS-USC-751X, P11467, P-10358, bis(7)-tacrine, HMR-2420, CP-126998, TV-3279, MSF, THA-C8, subergorgic acid, suberogorgin, SPH-1286, huperzine B (Hup B), pyridostigmine bromide (Ro-1-5130), huprine Y, coronaridine, RS-1233, kobophenol A, bis(12)-huperine, RS-1259, ITH-4012, TK-19, T-81, TH-171, TH-185, distigmine bromide (BC-51), (-)-9-dehydrogalanthaminium bromide, memoquin, scopoletin 7-O-beta-D-glucopyranoside (NSC-404560), scopolyn (SCN), scopoloside, BW-284c51, withaferin A (NSC-101088), withaferine (NSC-273757), (+)-corynoline, corynoline, (S)-(-)-oxypeucedanin, oxypeucedanin, (-)-voacangine, carbomethoxyibogaine, voacangine, dieckol, phlorofucofuroeckol (PFF), phlorofucofuroeckol A, (-)-3-0-acetylspectaline hydrochloride and rhaphiasaponin 1, or salts, free bases, racemates or enantiomers thereof, and they could be used for treating AD according to the invention.

As it is used in the present invention, "NMDA receptor antagonist" refers to a chemical compound inhibiting the reaction generated by the polysynaptic discharge of nociceptive primary afferent fibers. Memantine (3,5-dimethyltricyclo[3.3.1.13,7]decan-1-amine or 3,5-dimethyladamantane-1-amine) stands out among antagonists.

Other NMDA antagonists are nimodipine (3-(2-methoxyethyl)5-propan-2-yl 2,6-dimethyl-4-(3-nitrophenyl)-1,4-dihydropyridine-3,5-dicarboxylate), dizocilpine, AP-5 (2-amino-5-phosphonopentanoic acid), AP-7 (2-amino-7-phosphonoheptanoic acid), CHF 3381 (n-(2-indanyl)-glycinamide hydrochloride]) and ifenprodil (4-[2-(4-benzylpiperidin-1-yl)-1-hydroxypropyl]phenol) and they could be used for treating AD according to the invention.

As it is used in the present invention, "anti β-amyloid immunotherapy", refers to a therapy using the immune system for slowing or stopping the damages caused by β-amyloid accumulation. Said immunotherapy comprises the administration of anti beta-amyloid antibodies. Anti beta-amyloid antibodies useful for treatment according to the present invention include those which directly destroy the amyloid plaque (microglia are stimulated by immunization and devour the already previously formed amyloid plaques), those which capture the amyloid (formation of the antigen-antibody complex in peripheral blood sequesters the amyloid out of the brain and prevents deposition) or those inhibiting aggregation (formation of the antigen-antibody complex prevents the amyloid from aggregating in senile plaques). Alternatively, anti β-amyloid immunotherapy comprises the use of immunogenic compositions comprising one or several β-amyloid peptides (for example Aβ(1-5), Aβ(1-6), Aβ(1-12), Aβ(13-28), Aβ(25-35), Aβ(35-42), Aβ(33-42) and Aβ(33-40)) (the expression Aβ(X-Y) refers to a peptide derived from the beta-amyloid peptide consisting of the amino acids at position X to the amino acid at position Y). The beta-amyloid peptides can be administered either in the form of conjugates (for example, conjugated to KLH or an albumin) or in the form of a composition with an adjuvant (for example, Freund's complete adjuvant, Freund's incomplete adjuvant, QS21, aluminum hydroxide gel, MF59, calcium phosphate, liposyn, saponin, squalene, L121, monophosphoryl lipid A (MPL) in Emulsigen, polysorbate 80, cholera toxin (CT), LTK and LTK63). By way of illustrative example, a vaccine useful in the present invention would be the AN-1972 vaccine, which consists of ßA42 being an artificial copy of the human beta-amyloid protein, which when injected stimulates the immune system to clean abnormal deposits from the brain of sick rodents. This stimulates the formation of antibodies which adhere to the neuritic plaques to later be digested by the cellular elements of the immune system (microglia, astrocytes).

Alternatively, it is possible to use gamma secretase inhibitors, gamma secretase being an enzyme involved in β amyloid synthesis.

"Gamma secretase inhibitors" as used herein to a compound that inhibits the gamma secretase being an enzyme involved in β amyloid synthesis. Gamma secretase inhibitors useful in the present invention are, among others, LY-450139 (CAS no. 425386-60-3), also referred to as Semagacestat, DAPT (CAS no. 208255-80-5), CTS-21166 or MK 8931.

An "anti-tau therapy" would be analogous to anti-amyloid-beta immunotherapy against extracellular plaques. Such therapy includes inhibitors of tau kinases, enhancement of phosphatase activity to enhance microtubule stability, altering or blocking tau hyperphosphorylation, decreasing or inhibit tau aggregates and filament formation. In a preferred embodiment, the compound is an anti- tau antibody, such as those disclosed in Yanamandra K. et al., Annals of clinical and translational Neurology Volume 2, Issue 3, pages 278-288, March 2015

The terms described in detail in the context of the diagnostic method of the invention and their definitions and particularities apply equally to this aspect of the invention.

The invention is illustrated below based on the following examples provided by way of non-limiting illustration of the scope of the invention.

### Example 1

### Sample preparation

The blood plasma samples were analyzed by means Raman spectroscopy after extending the plasma on a 1cm² square surface CaF₂. After only 2 minutes, the sample is dry and ready to be analyzed.

### Raman Spectroscopy

Raman spectroscopy was performed using a BWS465-785S Raman spectrometer illuminated with a laser at 785 nm. Detection was performed with a 2048 pixels linear CCD and a Raman probehead. Spectral resolution was 4.5cm⁻¹ at 912nm, the spectrometer is coupled by fiber optics to a BAC 151B videomicroscope. A CFI LU Plan FLUOR EPI 100X Nikon objective was used allowing a laser spot of 26 µm diameter. Power on samples was controlled below thermal damage on the blood plasma. Spectra were calibrated using a NIST SRM2241 standard.

### Results

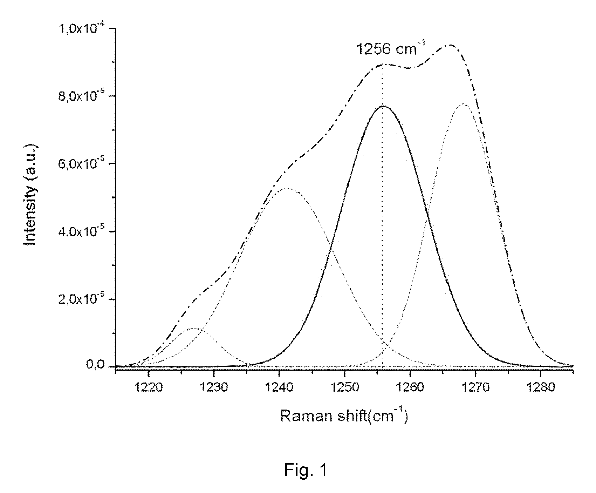

A detailed Raman study has been carried out in 250 patient samples in an ongoing multicenter study involving 14 different centers across Europe. With the Raman spectra obtained from the analyses, the inventors performed a normalization and a deconvolution of the spectra in order to obtain all the spectral features in the spectral region of interest (Fig. 1).

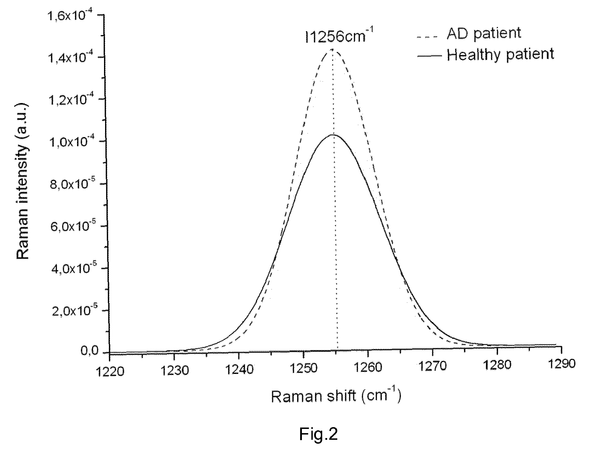

The classification of patients based on the spectroscopic parameter has been found to be significant. Figure 2 shows the difference in intensity of the band around 1256 cm⁻¹ between AD and healthy patients.

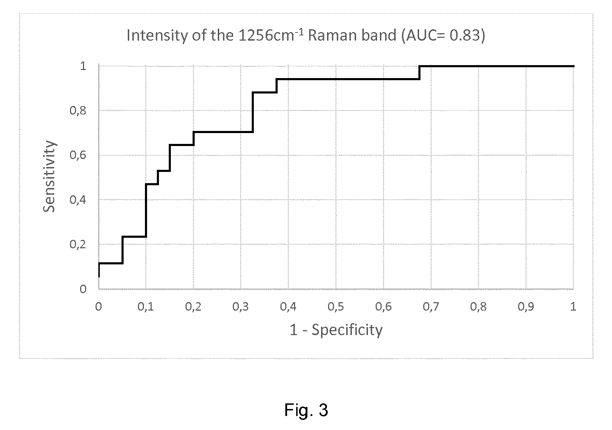

The spectroscopic parameter that allows the classification of patients with Alzheimer's disease with high probability is the intensity value of a Raman band at a frequency of around 1256 cm⁻¹ (Fig. 3).

## Claims

1. A vibrational spectroscopy method for diagnosing Alzheimer's disease in a subject comprising the steps of:
a) determining in a Raman spectrum of a biofluid from said subject or in a sample derived from said biofluid the intensity value of a Raman band at a frequency of around 1256 cm⁻¹; and
b) comparing the intensity value obtained in step a) with a reference value, wherein an increase in the value of said Raman band intensity with respect to the reference value is indicative that the subject suffers Alzheimer's disease.

2. The method according to claim 1 wherein the Raman spectrum is recorded using a near-infrared laser excitation line.

3. The method according to any one of claims 1 to 2, wherein the biofluid is plasma.

4. The method according to any of claims 1 to 3 wherein the sample derived from said biofluid is dehydrated plasma.

5. The method according to claim 4 wherein the dehydrated plasma is prepared by extending the plasma sample on a CaF₂ or ZnSe crystal or on a metallic support and allowing it to evaporate to dryness.

6. The method of claim 5 wherein the volume of the plasma sample is between 6 and 100 µL and/or wherein the evaporation to dryness is carried out at a temperature comprised between 10 and 25°C.

7. The method according to claim 6 wherein the sample is a blood plasma fraction and the volume which is analyzed is 10 µL.

8. The method according to any of claims 1 to 7 wherein the reference value is the intensity value of the Raman band at a frequency of around 1256 cm⁻¹ in a control patient.

9. The method according to any of claims 1 to 8 wherein the intensity value is obtained after normalizing the spectrum value with respect to the total area under the Raman curve.

10. An apparatus for plasma sample analysis comprising:
a) a receptacle for receiving a plasma sample,
b) at least one Raman spectrometer
c) a computer system comprising means for implementing a diagnostic method according to any of claims 1 to 9.

11. A computer program comprising a code suitable for performing the diagnostic methods according to any of claims 1 to 9.

12. A data carrier containing the computer program according to claim 11.

13. A therapy suitable for treating Alzheimer's disease for use in treating Alzheimer's disease in a subject wherein said subject has been selected by a diagnostic method according to any of claims 1 to 9.

14. The therapy for use according to claim 13 wherein said therapy is selected from the group consisting of a cholinesterase inhibitor, an N-methyl-D-aspartate (NMDA) receptor antagonist, anti β-amyloid immunotherapy, a gamma-secretase inhibitor, an anti-tau therapy and combinations thereof.
